# FASCICULE DE BREVET EUROPEEN

(11) **EP 3 830 542 B1**
(45) Date de publication et mention de la délivrance du brevet: **28.09.2022**
(21) Numéro de dépôt: 19744731.1
(22) Date de dépôt: 30.07.2019
(51) Int. Cl.: G01M 17/02, G01N 3/56, G01N 33/00, B60C 11/24

(54) **METHODE D'ANIMATION D'UN PNEUMATIQUE SUR UN VOLANT D'USURE**
VERFAHREN ZUR REIFENBETÄTIGUNG AUF EINEM VERSCHLEISSSCHWUNGRAD
METHOD FOR ACTUATING A TYRE ON A WEAR FLYWHEEL

(30) Priorité: 31.07.2018 FR 1857142
(43) Date de publication de la demande: 09.06.2021
(73) Titulaire: Compagnie Générale des Etablissements Michelin, 63000 Clermont-Ferrand (FR)
(72) Inventeur: CETTOUR-JANET, Dominique, 63040 Clermont-Ferrand Cedex 9 (FR)
(74) Mandataire: Roussy, Delphine
(86) Numéro de dépôt international: PCT/EP2019/070488
(87) Numéro de publication internationale: WO 2020/025606

(56) Documents cités:
- US-A1- 2005 066 719
- US-A1- 2009 012 763
- US-A1- 2018 201 077

## Description

L'invention s'intéresse au domaine des essais d'usure de pneumatiques, et en particulier aux essais d'usure réalisés sur des machines de roulage.

Les essais d'usure de pneumatiques sont réalisés pour une grande majorité d'entre eux sur des circuits routiers dûment répertoriés et sur lesquels des véhicules expérimentaux effectuent des parcours normalisés.

Ce mode d'évaluation reste toutefois très consommateur de ressources. Aussi, depuis de nombreuses années des protocoles d'essais sont développés pour réaliser ces essais sur des machines de roulage.

Toutefois, les protocoles développés par les différents laboratoires de test peinent à reproduire les conditions de roulage réelles et à proposer des classements fiables des pneumatiques les uns par rapport aux autres en regard de leurs performances à l'usure.

De plus, ces tests sont largement dépendants des types de véhicules et des caractéristiques de leurs suspensions avant et arrière, des charges supportées, du type de conduite, des vitesses de roulage, des circuits parcourus, de la nature des revêtements routiers rencontrés, des conditions atmosphériques régnant lors du test, etc...,.

A cet effet, les protocoles d'évaluation sur machine de test les plus performants utilisent les modèles physiques permettant de décrire les effets dynamiques du véhicule sur le pneumatique.

Ces modèles décrivent les équations reliant les vitesses et accélérations appliquées au centre de gravité du véhicule aux torseurs des efforts appliqués aux centres roues Fx, Fy, Fz, Cx, Cy, Cz. L'équilibre dynamique du véhicule est alors fonction de la commande de braquage et de la vitesse de translation véhicule imposée par le conducteur, résultant dans les angles de roulis et de tangage imposés par la construction mécanique des suspensions du véhicule, et conduisant vers les angles de dérive et de carrossage ainsi qu'aux charges supportées par chacune des roues du véhicule.

Ces équations utilisent pour décrire le comportement du pneumatique des modèles physiques connus, tel que, par exemple, le modèle mis au point par Hans Bastiaan Pacejka (Tyre modelling for use in vehicle dynamics studies, 1987 Jan. Society of Automotive Engineers, Warrendale, PA). Ces modèles non linéaires sont fondés sur des fonctions mathématiques ajustées sur une caractérisation expérimentale du pneumatique sous différentes allures de charge et d'angles de dérive et de carrossage, ainsi que sous glissement longitudinal telles que permises sur des machines de mesure de pneumatique du type Flat-Track que commercialisées par exemple par la Société MTS ou sur des tambours de roulage d'un diamètre suffisant pour que l'effet de la courbure du sol soit négligeable sur les forces et moments produits par le pneumatique comparativement à un sol plan. Il est également possible de mettre en œuvre une modélisation physique du pneumatique plus ou moins raffinée allant d'un découpage macro-structurel jusqu'à une description par Eléments Finis pour obtenir le jeu de caractéristiques décrivant le comportement dudit pneumatique.

De manière alternative, ces lois peuvent être mises au point à l'aide d'acquisitions expérimentales en instrumentant un véhicule en cours de roulage, ou plus simplement, en effectuant des mesures sur une plateforme dynamométrique telle que décrite par exemple dans la norme AMTI Model OR6-5-2000.

Un procédé pour prédire l'usure des pneus d'un véhicule est connu du document US 2005/066719 A1. Le procédé consiste à déterminer un ensemble de charges de pneus et utiliser l'ensemble de charges de pneus pour analyser l'usure des pneus. Le document US 2018/201077 A1 divulgue un procédé de génération d'historiques de charge de pneu à partir d'un modèle de pneu de base, d'un modèle de véhicule de base et de données de parcours d'essai simulé pour effectuer un test d'usure de pneu. Le document US 2009/012763 A1 divulgue une méthode et un système d'évaluation et de réglage des pneus comprenant un banc d'essai sur lequel sont montés un ou plusieurs pneus physiques. Un modèle de véhicule complet et une description de route sont utilisés avec le banc d'essai pour tester et évaluer le pneu comme cela serait fait sur une piste d'essai réelle.

La publication EP 1 354 184 divulgue un modèle expérimental pouvant répondre à cette préoccupation.

Selon cette publication, il est proposé de procéder aux étapes suivantes.

Une première étape consiste à caractériser le véhicule en mesurant, à l'aide d'une plateforme dynamométrique, les forces directionnelles Fx, Fy, Fz et les angles de dérive et de carrossage rencontrés par chacun des pneumatiques pendant ladite étape de caractérisation du véhicule au cours de laquelle on fait circuler le véhicule sur la plateforme à des allures différentes d'accélération, de vitesse de translation et de rayon de virage.

La deuxième étape du procédé selon EP 1 354 184 consiste à caractériser un parcours de test d'usure en mesurant les accélérations et les vitesses au centre de gravité du véhicule lorsque le véhicule roule sur le parcours test, préférentiellement identique au parcours test utilisé pour faire les tests en condition de roulage réel et sur lequel des résultats d'usure expérimentaux ont déjà été acquis. Cette deuxième étape peut être indépendante du véhicule et du train de pneumatiques retenu.

Au cours d'une troisième étape du procédé, les résultats expérimentaux acquis au cours de la première étape permettent de créer des lois expérimentales propres au véhicule et au train de pneumatiques, et reliant les trois forces directionnelles Fx, Fy et Fz, et l'angle de dérive appliquées au centre roue et rencontrés par chacun des pneumatiques, aux accélérations (Ax, Ay) et à la vitesse de translation (Vx) du véhicule, mesurées au centre de gravité.

En utilisant un modèle dynamique du véhicule, et après avoir qualifié la géométrie des trains roulants, associé à un modèle du pneumatique tel que celui développé par Hans Bastiaan Pacejka on peut, de manière équivalente, déterminer par calcul ces relations en s'affranchissant de la première étape.

Enfin, l'étape de test proprement dite consiste à monter une roue supportant le pneumatique à tester sur un volant, et à asservir la machine de test pour appliquer sur la roue les forces Fx, Fy, Fz et l'angle de dérive rencontrés par le pneumatique au cours du parcours de test, et calculés à partir des lois obtenues au cours de la troisième étape et des données d'accélération et de dérive acquises au cours de la deuxième étape. En faisant ainsi parcourir sur volant une distance similaire à celle réalisée par le véhicule sur le circuit d'usure de référence, on reproduit ainsi sur le pneu testé les performances en usure relevée sur ledit circuit.

Bien que permettant d'obtenir des résultats d'usure satisfaisants et proches des résultats obtenus sur le circuit de test par un véhicule, cette méthode présente toutefois l'inconvénient d'engager au cours de la première étape des plans expérimentaux lourds et onéreux. De manière similaire, la détermination par identification expérimentale (i.e modèle Pacejka) des lois dynamiques prenant en compte le fonctionnement du pneumatique est lourde et complexe et ne peut pas s'exécuter en temps réel. Il en est de même si on adopte une démarche de modélisation pure du pneumatique pour évaluer ces mêmes caractéristiques de fonctionnement.

L'objet de l'invention est de proposer une méthode de test d'usure de pneumatique permettant de simplifier la procédure décrite ci-dessus et d'alléger les coûts et les temps de calcul nécessaires à son exécution.

La méthode de test de l'usure de pneumatiques roulant sur un volant d'une machine de test comprenant au moins une paire de poste de mesure selon l'invention prévoit la mise en œuvre des étapes au cours desquelles :
- **Etape A :** à l'aide des données de construction d'un véhicule et d'un modèle d'équilibre dynamique on détermine les relations entre la vitesse de translation et les accélérations au centre de gravité du véhicule et les forces longitudinales, transversales et les variations de charges appliquées par chaque essieu sur un châssis du véhicule, la répartition des forces longitudinales et des variations de charges appliquées par le sol sur chacune des roues, et l'angle de carrossage de chacune des roues.
- **Etape B** : on mesure et on enregistre en continu la vitesse de translation et les accélérations au centre de gravité du véhicule lorsque le véhicule circule sur un parcours de test d'usure prédéterminé,
- **Etape C** : on dispose sur chacun des postes de mesure de la machine de test les deux pneumatiques appartenant à un même essieu et,
   ∘ à l'aide des relations déterminées à l'étape A et en fonction des valeurs de vitesse de translation et d'accélération longitudinale, transversale et verticale enregistrées à l'étape B, on détermine à chaque instant les valeurs de la force longitudinale, de la force transversale et de la force verticale relatives audit essieu, ainsi que les valeurs d'angle de carrossage, de charge et de forces longitudinales relatives à chacune des roues dudit essieu,
   ∘ on applique à chaque instant sur chacune des roues les valeurs d'angle de carrossage ainsi que les valeurs des forces longitudinales et verticales calculées précédemment et,
   ∘ on mesure les valeurs des forces transversales subies par chacune des roues dudit essieu et on fait varier l'angle de dérive de chacune des roues de sorte que la somme des forces transversales résultant de la mise en dérive des pneumatiques soit égale à chaque instant à la force transversale appliquée au centre de l'essieu et que la différence de dérive entre les deux roues respecte la variation de parallélisme calculée à partir des caractéristiques de l'essieu.

Il a été mis en évidence que les lois utilisées par les modèles d'équilibre dynamique des véhicules permettant de calculer les valeurs des angles de roulis et de carrossage ainsi que les valeurs de la force longitudinale, de la force transversale et de la force verticale appliquées audit essieu, ainsi que les valeurs de charge et d'accélération relatives à chacune des roues dudit essieu en fonction des valeurs d'accélération et de vitesse de translation au centre de gravité, peuvent être facilement accessibles à l'aide des données mécaniques et géométriques du véhicule. Ces lois peuvent par exemple être formulées à partir d'un modèle dit modèle quadricycle connu en soi.

A la différence des modèles permettant d'évaluer les forces transversales s'appliquant sur chacun des pneumatiques d'un même essieu, tel que le modèle développé par Hans Bastiaan Pacejka évoqué ci-dessus ou ses équivalents, et qui nécessitent de réaliser des mesures complexes ou d'introduire des lois non linéaires prenant en compte le comportement du pneumatique lorsque ce dernier est soumis à des variations de dérive, de charge, de carrossage et de glissement longitudinal, la méthode selon l'invention propose de s'affranchir de ces étapes en considérant que la force transversale appliquée à l'essieu est la résultante des forces latérales subies par chacun des pneumatiques formant un angle de dérive avec la direction longitudinale.

La machine de test est alors asservie pour aller « rechercher » l'angle de dérive à conférer aux pneumatiques de sorte que la somme des forces transversales mesurées sur chacune des roues soit égale à la force transversale appliquée à l'essieu tout en respectant les différences d'angles de braquage de chacune des roues imposées par la géométrie de l'essieu.

On parle alors d'un principe de fonctionnement connu sous l'appellation de « matériel dans la boucle » ou encore sous la dénomination anglo-saxonne « hardware in the loop ». En d'autres termes, la valeur de l'angle de dérive et des forces latérales n'étant pas connue individuellement pour chacun des pneumatiques de l'essieu, c'est l'asservissement de la machine qui détermine la valeur angulaire de la dérive de sorte que la somme des forces latérales subies par chacun des pneumatiques soit égale à la force latérale à l'essieu tout en garantissant l'écart de parallélisme entre les roues dudit essieu, ces deux termes étant facilement accessibles en utilisant le modèle quadricycle.

La méthode de test d'usure permet ainsi de s'affranchir de la caractérisation expérimentale et de la modélisation permettant de décrire le pneumatique et offre de plus la possibilité, une fois acquises les données de construction du véhicule, de tester pour ce véhicule des trains de pneumatiques quelconques.

La méthode selon l'invention permet également de prendre en compte l'évolution des propriétés physiques de chacun des pneumatiques suivant la progression de leur degré d'usure au cours du test. En effet, la machine de test adapte automatiquement les angles de dérive de chacun des pneumatiques de l'essieu considéré pour obtenir la valeur désirée de la force transversale totale appliquée à l'essieu. A l'inverse dans une démarche de caractérisation expérimentale ou de modélisation des caractéristiques du pneumatique, il faudrait dans l'idéal faire évoluer ces caractéristiques au cours du test en fonction de l'usure pour ajuster en continu l'animation du pneumatique sur la machine de test, ce qui devient très lourd en pratique.

Les résultats d'usure s'approchent alors fidèlement des résultats obtenus en faisant rouler le véhicule sur le parcours de test sélectionné.

Les tests d'usure peuvent alors être obtenus à des conditions économiques très favorables et dans des délais réduits.

La méthode de test selon l'invention peut aussi comprendre isolément, ou en combinaison, l'exécution des actions suivantes :
- La valeur de la force longitudinale appliquée par chaque essieu sur le châssis résulte du modèle d'équilibre dynamique du véhicule sous l'effet de l'accélération longitudinale, des données de construction du véhicule, de la vitesse de translation et des efforts de traction fournis par le moteur.
- Les forces longitudinales appliquées par le sol sur chacune des roues d'un même essieu sont égales à chaque instant
- La répartition entre les forces longitudinales appliquées par le sol à chacune des quatre roues résulte d'une loi spécifique de commande introduite dans le modèle d'équilibre dynamique du véhicule.

- La somme des variations de charges appliquées par le sol sur chacune des roues d'un même essieu résulte du modèle d'équilibre dynamique du véhicule en tangage sous l'effet de la vitesse, de l'accélération longitudinale et des données de construction du véhicule.
- La différence des variations de charges appliquées par le sol sur chacune des roues d'un même essieu résulte du modèle d'équilibre dynamique du véhicule en roulis sous l'effet de l'accélération transversale et des données de construction du véhicule
- La valeur de la force transversale appliquée par chaque essieu sur le châssis résulte du modèle d'équilibre dynamique du véhicule en lacet sous l'effet de l'accélération transversale et des données de construction du véhicule.
- L'angle de carrossage de chaque roue résulte du modèle d'équilibre dynamique du véhicule en roulis sous l'effet de l'accélération transversale et des données de construction du véhicule.
- On corrige la valeur de l'angle de carrossage de sorte que cet angle résulte du modèle d'équilibre dynamique corrigé de l'effet des variations élastiques de carrossage induites par les efforts transversaux mesurés à chaque instant sur le volant.
- On fait varier les angles de dérive de chacune des roues d'un même essieu, de sorte que la différence entre les angles de dérive de chacune des roues soit égale à une valeur connue.
- La valeur de la différence entre les angles de dérive de chacune des roues d'un même essieu est nulle à chaque instant.
- On corrige la valeur de la différence entre les angles de dérive de chacune des roues d'un même essieu de sorte que cette différence résulte du modèle d'équilibre dynamique corrigé de l'effet de la valeur des variations élastiques de braquage induites par les variations de charge et les efforts longitudinaux calculés, et par les efforts transversaux mesurés à chaque instant sur le volant.
- La machine de test est équipée d'une pluralité de paires de postes de mesure de sorte que l'on peut effectuer :
   ∘ des tests de trains de pneumatiques montés sur les essieux avant et arrière d'un même véhicule,
   ∘ des tests de trains de pneumatiques différents appartenant à un même essieu d'un même véhicule,
   ∘ des tests de trains de pneumatiques montés sur le même essieu d'un même véhicule comportant des réglages différents des données de construction du véhicule,
   ∘ des tests de trains de pneumatiques identiques (ou différents) montés sur le même essieu de véhicules différents.
   ∘ des tests de trains de pneumatiques différents montés sur le même essieu de véhicules différents.

L'invention sera mieux comprise à la lecture des figures annexées, qui sont fournies à titre d'exemples et ne présentent aucun caractère limitatif, dans lesquelles :
- La figure 1 représente une vue schématique d'un véhicule et des résultantes des forces appliquées sur chacun des pneumatiques.
- La figure 2 est une vue schématique d'une machine de roulage.
- La figure 3 est un diagramme illustrant les interactions entre les différentes forces prises en compte dans le modèle d'équilibre dynamique du véhicule.

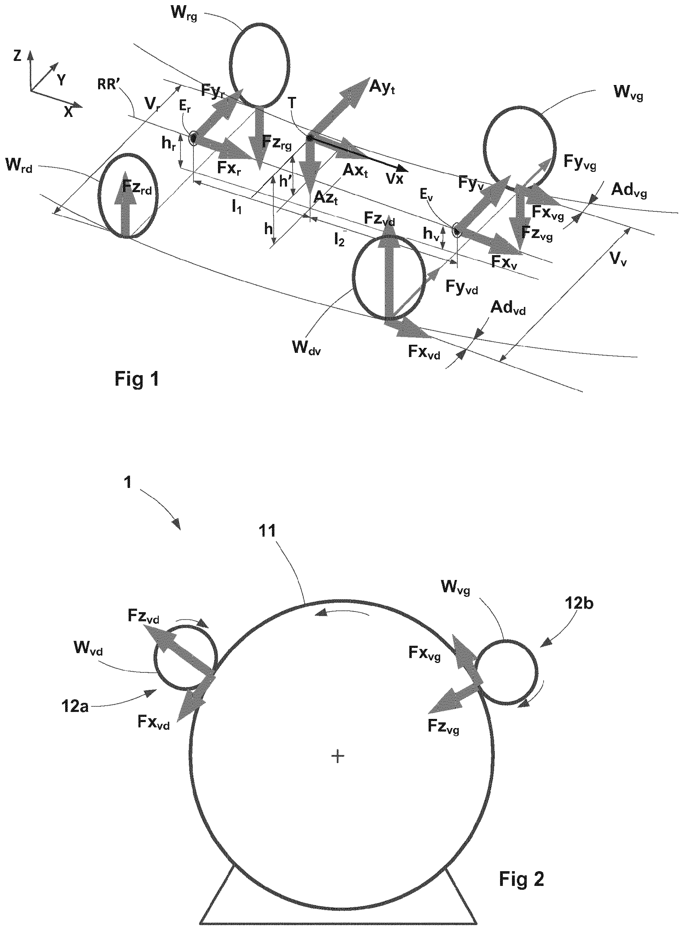

En référence à la figure 1, le véhicule est ici schématisé par la roue avant gauche W_{vg} intérieure au virage, la roue avant droite W_{vd} extérieure au virage, montées sur l'essieu avant Eᵥ, et par la roue arrière gauche W_{rg} intérieure au virage et par la roue arrière droite W_{rd} extérieure au virage, montées sur l'essieu arrière Eᵣ. Il va de soi que les roues droites et gauches peuvent être alternativement positionnées à l'intérieur ou à l'extérieur du virage selon l'allure du véhicule.

Les mêmes lois et les mêmes phénomènes s'appliquent sur l'essieu avant Eᵥ que sur l'essieu arrière Eᵣ. Aussi, pour une meilleure compréhension, il sera fait référence à l'avant du véhicule en mettant un indice « v » aux forces ou aux angles observés sur l'essieu avant Eᵥ et un indice « r » aux forces ou aux angles observés sur l'essieu arrière. L'absence d'indice faisant alors indistinctement référence aux forces ou aux angles observés sur l'essieu avant ou sur l'essieu arrière. De même, lorsque cela sera utile pour la compréhension de l'invention, il sera fait mention du côté droit et du côté gauche du véhicule en annotant respectivement les forces ou les angles respectivement d'un indice « d » ou « g ». Enfin, les valeurs des accélérations au centre de gravité T sont repérées à l'aide d'un indice « t ».

Le véhicule est alors inscrit dans son repère dans lequel l'axe X représente l'axe longitudinal, l'axe Y représente l'axe transversal, et l'axe Z représente l'axe vertical.

Le centre de gravité T du véhicule est disposé à une hauteur h du sol et à une hauteur h' de l'axe de roulis RR' passant par l'essieu avant Eᵥ et par l'essieu arrière Eᵣ. Le centre de gravité T est distant respectivement de l'essieu arrière d'une valeur l₁ et de l'essieu avant d'une valeur l₂. Les centres de roulis de l'essieu avant Eᵥ et de l'essieu arrière Eᵣ sont disposés respectivement à une hauteur hᵥ et hᵣ du sol. Vv et Vr représentent la largeur des voies avant et arrière.

Ces valeurs géométriques, ainsi que les caractéristiques élastocinématiques de construction du véhicule, sont introduites dans le modèle d'équilibre dynamique. A ces données mécaniques sont rajoutées la valeur de répartition de la masse du véhicule, les valeurs des angles de pince (Ap) ou des angles de carrossage statique (Acs) conférées à chacune des roues, des éléments permettant de décrire la raideur et la dynamique des éléments de suspension et des barres antiroulis, des données relatives aux coefficients aérodynamiques du véhicule ainsi que des données relatives à la résistance à l'avancement des pneumatiques

L'ensemble des données de construction du véhicule est repéré sous la dénomination commune Dc.

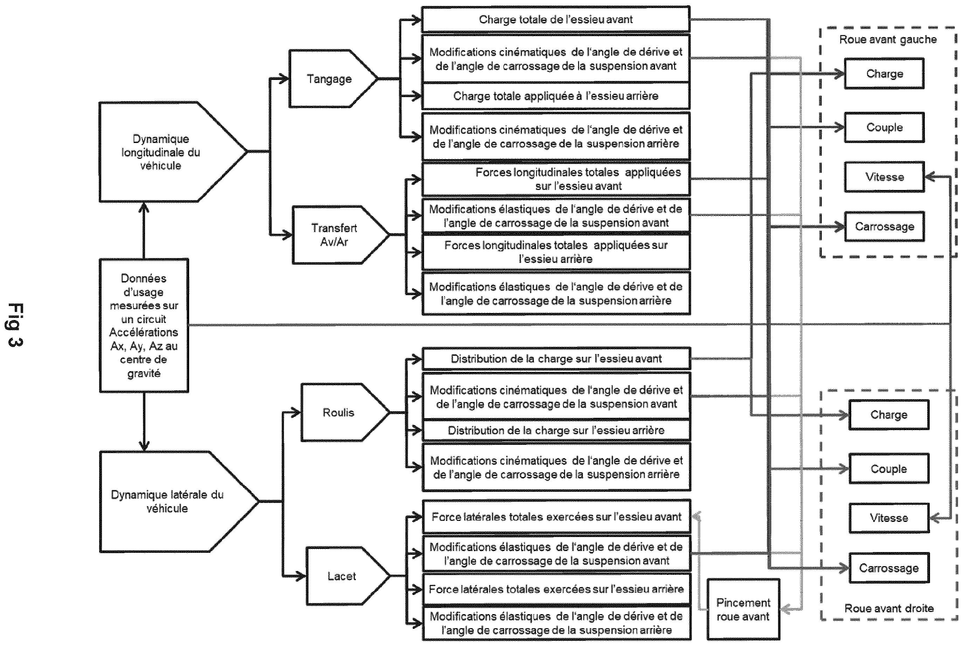

En référence à la figure 3, le modèle d'équilibre cinématique et dynamique dit « quadricycle » du véhicule va alors permettre de calculer les valeurs des forces exercées sur l'essieu avant et sur l'essieu arrière lorsque le véhicule circule à une vitesse de translation Vx et subit des accélérations longitudinales Axₜ, transversales Ayₜ et verticale Azₜ au centre de gravité T.

A l'aide des équations décrivant la dynamique latérale et longitudinale du véhicule résultant des forces appliquées au centre de gravité on détermine :
- une distribution des variations de charges appliquées sur chacun des essieux Fzᵥ, Fzᵣ avant ou arrière ainsi que les modifications dynamiques de l'angle de dérive Ad et de l'angle de carrossage Ac des trains avant et arrière en fonction du tangage,
- une distribution des forces longitudinales appliquées sur chacun des essieux avant ou arrière Fxᵥ, Fxᵣ, ainsi que les modifications élastiques de l'angle de dérive Ad et de l'angle de carrossage Ac des trains avant et arrière en fonction des transferts de charge entre essieux avant et arrière,
- une distribution des variations de charges Fz_{vg}, Fz_{vd}, Fz_{rg}, Fz_{rd} sur chacune des roues Wv_{g}, Wv_{d}, Wr_{g}, Wr_{d} de l'essieu avant ou arrière ainsi que les modifications dynamiques de l'angle de dérive Ad et de l'angle de carrossage Ac en fonction du roulis.
- Les forces latérales Fyᵥ, Fyᵣ totales exercées sur l'essieu avant ou arrière ainsi que les modifications élastiques de l'angle de dérive Ad et de l'angle de carrossage Ac des trains avant et arrière en fonction de l'équilibre dynamique en lacet.

On en déduit alors les valeurs de charge, de couple (freineur ou moteur), de vitesse de translation et d'angle de carrossage à appliquer sur chacune des roues.

Au cours de **l'étape A,** on utilise donc le modèle dynamique du véhicule pour calculer les valeurs des forces et des angles appliquées à l'essieu.

La force longitudinale appliquée par l'essieu sur le châssis, *Fx = f₁*(*Axₜ,Vx,Dc*)*,* est la résultante des forces de freinage et d'accélération Axₜ, ainsi que des forces nécessaires pour s'opposer à la résistance aérodynamique de l'air et aux forces de frottement.

La différence Fx_{g}-Fx_{d} des forces longitudinales appliquées par le sol à chaque roue peut être considérée comme nulle en faisant l'hypothèse que le différentiel disposé sur chacun des essieux ou que le système de freinage, en dehors des fonctions de type ABS ou ESP, assurent un partage égal de ces forces. Dans le cas contraire, le modèle d'équilibre dynamique du véhicule devra comporter une loi spécifique de commande décrivant le mode de répartition des forces longitudinales (Fx_{vg}, Fx_{vd}, Fx_{rg}, Fx_{rd}) entre les quatre roues dudit véhicule, à l'image de ce qui est pratiqué sur des véhicules à quatre roues motrices, ou encore dans la répartition avant /arrière des efforts de freinage ou des efforts moteurs

La somme des variations de charges appliquées par le sol sur chacune des roues d'un même essieu, est la résultante de la répartition des variations de charges dans le véhicule et de l'équation d'équilibre du véhicule en tangage sous l'effet de la vitesse Vx et de l'accélération longitudinale Axₜ : *Fz_{g}* + *F_{Zd} = f₂(Axₜ,Vx,Dc)*

La différence de variation de charge appliquées par le sol sur chacune des roues de ce même essieu résulte des données Dc de construction du véhicule et de l'équation d'équilibre en roulis sous l'effet de l'accélération transversale Ayₜ : *Fz_{g}* - *Fz_{d} = f₃(Ayₜ,Dc) .*

La force transversale Fy appliquée par chaque essieu sur le châssis est la résultante des données Dc de construction du véhicule et de l'accélération transversale liée aux forces centripètes : *Fy = f₄(Ayₜ,Dc).*

L'angle de carrossage résulte également de l'équation d'équilibre en roulis sous l'effet de l'accélération transversale Ayₜ additionné des valeurs de carrossage statique Acs qui font partie des données de construction Dc du véhicule *: Ac = f*₅*(Ayₜ,Dc).*

On observera ici que la mesure en temps réel des forces transversales Fy_{g} et Fy_{d} appliquées par le sol sur la roue gauche ou la roue droite d'un même essieu et observées sur le volant de mesure 11 pendant l'étape C permet d'affiner le mode d'obtention de la valeur des angles de carrossage.

En effet, sous l'effet des forces transversales Fy_{g} et Fy_{d}, les angles de carrossage appliqués à chacune des roues sont à corriger pour tenir compte des déformations élastiques s'appliquant sur l'essieu.

Pour affiner le calcul, on peut introduire à partir des valeurs mesurées Fy_{g} et Fy_{d} sur le volant 11 une boucle interactive secondaire de sorte que la loi permettant de déterminer l'angle de carrossage devient, à titre d'exemple, pour la roue avant droite *Ac_{vd} = f'₅(Ayₜ,Fy_{vd},Fy_{vg},Dc)* et, pour la roue avant gauche *Ac_{vg} = f'*₅*(Ayₜ,Fy_{vd},Fy_{vg},Dc).*

La différence entre les angles de dérive appliqués sur la roue droite W_{d} et sur la roue gauche W_{g} est généralement issue des données de construction du véhicule, et peut être obtenue à l'aide d'une loi δ(Ad) qui est fonction des réglages statiques de pince ou d'ouverture et des règles de braquage imposées mécaniquement à chacune des voies avant ou arrière, corrigée des variations dynamiques liées aux mouvements de tangage et de roulis appliqués sur les roues : δ(*Ad*) *= f₆(Dc).*

Pour les mêmes raisons que celles qui ont été explicitées ci-dessus, il peut s'avérer utile de prendre en compte les déformations élastiques du train avant (ou du train arrière) engendrées par les efforts longitudinaux Fx_{vg}, F_{Xvd} appliqués par le sol sur les roues avant gauche et droite et les variations de charge verticale Fz_{vg}, Fz_{vd} appliquées par le sol sur les roues avant gauche et droite, et calculés à l'aide du modèle d'équilibre dynamique, ainsi que par les forces transversales Fy_{vg}, Fy_{vd} appliquées par le sol sur les roues avant gauche et droite mesurées sur le volant en temps réel au cours de l'étape C. La différence entre les angles de dérive δ(Ad) devient alors (par exemple pour les roues avant) du type δ(Ad) = *f*'*₆(Dc, Fx_{vg},Fx_{vd},Fy_{vg},Fy_{vd},Fz_{vg},Fz_{vd}).*

On observe que ces lois, spécifiques à un véhicule, ne dépendent pas de la nature du pneumatique et peuvent être mises en œuvre sans qu'il soit nécessaire de disposer d'un modèle de fonctionnement du pneumatique.

**L'étape B** du procédé consiste à collecter des données de vitesse de translation et d'accélération représentatives d'un roulage en condition réelle sur un circuit représentatif des conditions d'usure rencontrées par un pneumatique lors de son usage sur un véhicule correctement réglé conduit par un conducteur appliquant le niveau de sévérité de conduite visé par le « standard » du test.

Ce parcours comprend donc des tronçons de roulage sinueux, des parties urbaines, et des portions d'autoroute comportant des revêtements standards. De même, les conditions de roulage se font sur sol sec en veillant à exercer des freinages et des accélérations correspondant au niveau de sévérité de conduite « standard », tout en respectant les limites de vitesse imposées.

Un enregistreur, disposé au centre de gravité T du véhicule, enregistre en continu et en temps réel les données de vitesse Vx et d'accélération Axₜ, Ayₜ, Azₜ, subies par le véhicule tout au long du parcours de test.

Ces données enregistrées sont spécifiques du parcours de test, du niveau de sévérité « standard » et sont indépendantes du type de véhicule utilisé pour l'enregistrement.

**L'étape C** de la mise en œuvre du procédé selon l'invention est l'étape de test proprement dite. La machine de test roulage (1) telle qu'illustrée à la figure 2 comprend un volant 11 entrainé en rotation par un moteur (non représenté). La machine comporte au moins deux postes de mesure (12a, 12b) montés chacun sur un bras instrumenté (non représentés) supportant deux moyeux destinés à recevoir chacun une roue sur laquelle est monté un pneumatique et représentant la roue droite W_{d} et la roue gauche W_{g} d'un même essieu.

Il est tout à fait possible et de manière équivalente de réaliser les tests selon l'invention sur une machine de test de type flat-track du type de celle évoquée ci-dessus.

On observera ici que, compte tenu de ce qui précède, le volant 11 de la machine de test 1 peut être équipé d'une pluralité de paires de postes de mesure (12a, 12b). Cet arrangement offre alors une multitude de possibilités de tests différents.

On pourra par exemple réaliser des tests de trains de pneumatiques montés sur les essieux avant et arrière d'un même véhicule, ou encore tester des trains de pneumatiques différents appartenant à un même essieu,

Il est également possible de réaliser des tests de trains de pneumatiques montés sur le même essieu d'un même véhicule comportant des réglages différents des données élastocinématiques de construction Dc, ou de tester des trains de pneumatiques identiques ou différents montés sur le même essieu de véhicules différents.

La surface circonférentielle du volant 11 comporte un revêtement spécialement étudié pour reproduire fidèlement la granulométrie des revêtements routiers communément rencontrés sur le circuit de test d'usure. Ces revêtements peuvent être changés en cours de test pour s'approcher plus fidèlement de la réalité.

Chaque poste de mesure (12a, 12b) est équipé de moyens permettant de faire varier la flèche verticale du pneu, assimilée ici à la charge Fz portée par le pneumatique, les couples appliqués à la roue et simulant les phases d'accélération ou de freinage, l'angle de dérive (Ad_{g}, Ad_{d}) et l'angle de carrossage (Ac_{d}, Ac_{g}) de chacune des roues, ainsi que des moyens permettant de mesurer en continu la valeur des efforts (Fx_{d} ,Fy_{d}, Fz_{d}) ou (Fx_{g} ,Fy_{g}, Fz_{g}) appliqués par le sol sur chacune des roues. La vitesse de rotation du volant 11° est asservie de façon à représenter la vitesse Vx d'avance du véhicule.

A l'aide des lois f₁, f₂, f₃, f₄, f₅, f₆ déterminées à l'étape A, et en fonction des valeurs de vitesse Vx et d'accélération longitudinale, transversale et verticale Axₜ, Ayₜ, Azₜ enregistrées à l'étape B, on détermine à chaque instant les valeurs d'angle de carrossage Ac, les valeurs de la force longitudinale Fx, de la force transversale Fy et de la force verticale Fz relatives audit essieu E, ainsi que les valeurs de charge Fz_{g}, Fz_{d} et d'efforts longitudinaux Fx_{g}, Fx_{d} relatives à chacune des roues W_{g}, W_{d} de l'essieu considéré.

On applique en continu et à chaque instant sur chacune des roues les valeurs d'angle de carrossage Ac ainsi que les valeurs de force longitudinale et verticale Fx_{g}, Fx_{d}, et Fz_{g}, Fz_{d} subies par chacune des roues lorsque le véhicule circule le long du parcours de test d'usure. On reproduit ainsi sur une machine de roulage, et en temps réel, les conditions de roulage observées sur le circuit de test.

Seul l'angle de dérive appliqué à chacune des roues reste à déterminer.

Pour ce faire, on mesure les valeurs des forces transversales Fy_{g}, Fy_{d} subies par chacune des roues W_{g}, W_{d} et on fait varier l'angle de dérive Ad_{g}, Ad_{d} de chacune des roues de sorte que la somme des forces transversales Fy_{g}+Fy_{d} résultant de la mise en dérive des pneumatiques soit égale à chaque instant à la force transversale Fy appliquée au centre de l'essieu E considéré.

Simultanément, on asservit la différence entre les angles de dérive de la roue droite et de la roue gauche (Ad_{d}-Ad_{g} = δ(Ad)) de manière à tenir compte de la variation de parallélisme induite par la dynamique de roulis et de tangage du châssis.

Comme on l'a vu plus haut, cette différence d'angle de braquage est liée à la construction du train avant ou arrière. Dans les cas les plus simples, cette différence peut être nulle à tout instant ou intégrer la cinématique du train considéré ou, plus généralement prendre en compte les déformations élastiques engendrées par les forces longitudinales, verticales et transversales appliquées par le sol sur les roues.

Ainsi, l'asservissement de la machine de test « pilote » l'angle de dérive de chacune des roues jusqu'à ce que la valeur de la somme des forces transversales Fy_{g}+Fy_{d} mesurées sur chacun des postes12a et 12b de la machine supportant respectivement la roue droite W_{d} et la roue gauche W_{g} soit égale à la force Fy à cet instant.

Les forces transversales Fy_{d} et Fy_{g} engendrées par chacun des pneumatiques sur le volant 11 sous l'effet des angles de dérive et de carrossage dans les conditions de charge Fz_{d} et Fz_{g}, et qui obéissent naturellement aux lois non linéaires formant le modèle dynamique du pneumatique, sont alors reproduites avec des valeurs représentatives des conditions réelles de roulage.

La méthode faisant l'objet de la description ci-dessus permet de s'affranchir des calculs complexes liés à la détermination des forces transversales, et de reproduire de manière fiable et précise sur une machine de test les conditions de roulage en vue de pratiquer un test d'usure des pneumatiques d'un véhicule.

### NOMENCLATURE

- 1: Machine de test de test d'usure.
- 11: Volant.
- 12a, 12b: Postes de mesure des deux pneumatiques d'un même essieu.
- W_{vg}: Roue avant gauche (intérieure au virage).
- W_{vd}: Roue avant droite (extérieure au virage).
- W_{rg}: Roue arrière gauche (intérieure au virage).
- W_{rd}: Roue arrière droite (extérieure au virage).
- T: Centre de gravité du véhicule.
- h: Hauteur du centre de gravité par rapport au sol.
- h': Hauteur du centre de gravité par rapport à l'axe de roulis du véhicule.
- I₁: distance entre le centre de gravité et l'essieu arrière.
- I2: Distance entre le centre de gravité et l'essieu avant.
- Axₜ: Accélération longitudinale au centre de gravité du véhicule.
- Ayₜ: Accélération transversale au centre de gravité du véhicule.
- Azₜ: Accélération verticale au centre de gravité du véhicule.
- Vx: Vitesse de translation.
- Eᵥ: Essieu avant.
- hᵥ: Hauteur du centre de roulis de l'essieu avant par rapport au sol.
- Vᵥ: Largeur de la voie avant.
- Fxᵥ: Force longitudinale appliquée par l'essieu avant sur le châssis.
- Fx_{vg}: Force longitudinale appliquée par le sol sur la roue avant gauche.
- Fx_{vd}: Force longitudinale appliquée par le sol sur la roue avant droite.
- Fyᵥ: Force transversale appliquée par l'essieu avant sur le châssis.
- Fy_{vg}: Force transversale appliquée par le sol sur la roue avant gauche.
- Fy_{vd}: Force transversale appliquée par le sol sur la roue avant droite.
- Fz_{vg}: Variation de charge verticale appliquée par le sol sur la roue avant gauche.
- Fz_{vd}: Variation de charge verticale appliquée par le sol sur la roue avant droite.
- Ad_{vg}: Angle de dérive de la roue avant gauche.
- Ad_{vd}: Angle de dérive de la roue avant droite.
- Eᵣ: Essieu arrière.
- hᵣ: Hauteur du centre de roulis de l'essieu arrière par rapport au sol.
- Vᵣ: Largeur de la voie arrière.
- Fxᵣ: Force longitudinale appliquée par l'essieu arrière sur le châssis.
- Fyᵣ: Force transversale appliquée par l'essieu arrière sur le châssis.
- Fz_{rg}: Variation de charge verticale appliquée par le sol sur la roue arrière gauche.
- Fz_{rd}: Variation de charge verticale appliquée par le sol sur la roue arrière droite.
- RR': Axe de roulis.
- Dc: Données de construction élastocinématiques du véhicule.

## Revendications

1. Méthode de test de l'usure de pneumatiques roulant sur un volant (11) d'une machine de test (1) comprenant au moins une paire de poste de mesure (12a, 12b), au cours de laquelle on exécute les étapes suivantes :
- Etape A : à l'aide des données de construction d'un véhicule (Dc) et d'un modèle d'équilibre dynamique on détermine les relations (f₁, f₂, f₃, f₄, f₅, f₆) entre la vitesse de translation (Vx) et les accélérations (Axₜ, Ayₜ, Azₜ) au centre de gravité du véhicule (T), et les forces longitudinales (Fx), transversales (Fy) et les variations de charges (Fz) appliquées par chaque essieu sur le châssis du véhicule, la répartition des forces longitudinales (Fx_{vg}, Fx_{vd}, Fx_{rg}, Fx_{rd}) et des variations de charges (Fz_{vg}, Fz_{vd}, Fz_{rg}, Fz_{rd}) appliquées par le sol sur chacune des roues, et l'angle de carrossage (Ac) de chacune des roues.
- Etape B : on mesure et on enregistre en continu la vitesse de translation (Vx) et les accélérations (Axₜ, Ayₜ, Azₜ) au centre de gravité (T) du véhicule lorsque le véhicule circule sur un parcours de test d'usure prédéterminé,
- Etape C : on dispose sur chacun des postes de mesure (12a, 12b) de la machine de test (1) les deux pneumatiques appartenant à un même essieu et,
∘ à l'aide des relations (f₁, f₂, f₃, f₄, f₅, f₆) déterminées à l'étape A et en fonction des valeurs de vitesse de translation (Vx) et d'accélération longitudinale (Axₜ), transversale (Ayₜ) et verticale (Azₜ) enregistrées à l'étape B, on détermine à chaque instant les valeurs de la force longitudinale (Fx), de la force transversale (Fy) et de la variation de charge (Fz) appliquées par l'essieu (E) sur le châssis du véhicule, ainsi que les valeurs d'angle de carrossage (Ac), de variations de charges (Fz_{g}, Fz_{d}) et de forces longitudinales (Fx_{g}, Fx_{d}) appliquées par le sol sur chacune des roues (W_{g}, W_{d}) dudit essieu (E),
∘ on applique à chaque instant sur chacune des roues (W_{g}, W_{d}) les valeurs d'angle de carrossage (Ac) ainsi que les valeurs des forces longitudinales et verticales (Fx_{g}, Fx_{d}, et Fz_{g}, Fz_{d}) calculées précédemment et,
∘ on mesure les valeurs des forces transversales (Fy_{g}, Fy_{d}) subies par chacune des roues (W_{g}, W_{d}) dudit essieu (E) et on fait varier l'angle de dérive (Ad) de chacune des roues de sorte que la somme des forces transversales (Fy_{g}+Fy_{d}) résultant de la mise en dérive des pneumatiques soit égale à chaque instant à la force transversale (Fy) appliquée au centre de l'essieu (E) et que la différence de dérive (δ(Ad)) entre les deux roues respecte la variation de parallélisme calculée à partir des caractéristiques de l'essieu.

2. Méthode de test selon la revendication 1, dans laquelle la valeur de la force longitudinale appliquée par chaque essieu sur le châssis résulte du modèle d'équilibre dynamique du véhicule sous l'effet de l'accélération longitudinale (Axₜ), des données (Dc) de construction du véhicule, de la vitesse de translation (Vx) et des efforts de traction fournis par le moteur (*Fx* = *f₁(Axₜ,Vx,Dc)*).

3. Méthode de test selon la revendication 2, dans laquelle les forces longitudinales appliquées par le sol sur chacune des roues d'un même essieu sont égales (Fx_{g}=Fx_{d}) à chaque instant.

4. Méthode de test selon la revendication 2 ou la revendication 3, dans laquelle la répartition entre les forces longitudinales appliquées par le sol à chacune des quatre roues (Fx_{vg}, Fx_{vd}, Fx_{rg}, Fx_{rd}) résulte d'une loi spécifique de commande introduite dans le modèle d'équilibre dynamique du véhicule.

5. Méthode de test selon l'une quelconque des revendications précédentes, dans laquelle la somme des variations de charges (Fz_{g}+Fz_{d}) appliquées par le sol sur chacune des roues (W_{g}, W_{d}) d'un même essieu résulte du modèle d'équilibre dynamique du véhicule en tangage sous l'effet de la vitesse (Vx), de l'accélération longitudinale (Axₜ) et des données (Dc) de construction du véhicule (*Fz_{g}* + *Fz_{d}* = *f₂(Axₜ,Vx,Dc*))*.*

6. Méthode de test selon l'une quelconque des revendications précédentes dans laquelle la différence des variations de charges (Fz_{g}-Fz_{d}) appliquées par le sol sur chacune des roues (W_{g}, W_{d}) d'un même essieu résulte du modèle d'équilibre dynamique du véhicule en roulis sous l'effet de l'accélération transversale (Ayₜ) et des données (Dc) de construction du véhicule *(Fz_{g}* - *F_{Zd} = f₃(Ayₜ,Dc)).*

7. Méthode de test selon l'une quelconque des revendications précédentes, dans laquelle la valeur de la force transversale (Fy) appliquée par chaque essieu sur le châssis résulte du modèle d'équilibre dynamique du véhicule en lacet sous l'effet de l'accélération transversale (Ayₜ) et des données (Dc) de construction du véhicule *(Fy = f_{A}(Ayₜ,Dc)).*

8. Méthode de test selon l'une quelconque des revendications précédentes dans laquelle l'angle de carrossage (Ac) de chaque roue résulte du modèle d'équilibre dynamique du véhicule en roulis sous l'effet de l'accélération transversale (Ayₜ) et des données de construction (Dc) du véhicule *(Ac = f₅(Ayₜ,Dc)).*

9. Méthode de test selon la revendication 8, dans laquelle on corrige la valeur de l'angle de carrossage (Ac) de sorte que cet angle résulte du modèle d'équilibre dynamique corrigé de l'effet des variations élastiques de carrossage induites par les efforts transversaux (Fy_{g} et Fy_{d}) mesurés à chaque instant sur le volant *(AC_{vd} = f'₅(Ayₜ,Fy_{vd},Fy_{vg},Dc)* et *Ac_{vg} = f'*₅*(Ayₜ,Fy_{vg},Fy_{vd},Dc)).*

10. Méthode de test selon l'une quelconque des revendications précédentes, dans laquelle on fait varier les angles de dérive de chacune des roues d'un même essieu, de sorte que la différence (Ad_{g}-Ad_{d}) entre les angles de dérive de chacune des roues soit égale à une valeur (δ(Ad)) connue *(δ(Ad) = f₆(Dc)).*

11. Méthode de test selon la revendication 10, dans laquelle la valeur de la différence entre les angles de dérive de chacune des roues d'un même essieu (δ(Ad)) est nulle à chaque instant.

12. Méthode de test selon la revendication 10, dans laquelle on corrige la valeur de la différence entre les angles de dérive de chacune des roues d'un même essieu (δ(Ad)) de sorte que cette différence (Ad_{g}-Ad_{d}) résulte du modèle d'équilibre dynamique corrigé de l'effet de la valeur des variations élastiques de braquage induites par les variations de charge (Fz_{vg}, Fz_{vd}) et les efforts longitudinaux (Fx_{vg}, F_{Xvd}) calculés, et par les efforts transversaux (Fy_{g} et Fy_{d}) mesurés à chaque instant sur le volant (*δ*(*Ad*) = *f'₆(Dc, Fx_{vg},Fx_{vd},Fy_{vg},Fy_{vd},Fz_{vg},Fz_{vd}).*

13. Méthode de test selon l'une quelconque des revendications précédentes dans laquelle la machine de test (1) est équipé d'une pluralité de paires (12a, 12b) de postes de mesure et dans laquelle on effectue :
- des tests de trains de pneumatiques montés sur les essieux avant et arrière d'un même véhicule,
- des tests de trains de pneumatiques différents appartenant à un même essieu d'un même véhicule,
- des tests de trains de pneumatiques montés sur le même essieu d'un même véhicule comportant des réglages différents des données de construction du véhicule Dc,
- des tests de trains de pneumatiques identiques montés sur le même essieu de véhicules différents,
- des tests de trains de pneumatiques différents montés sur le même essieu de véhicules différents.

## Patentansprüche

1. Testverfahren für den Verschleiß von Reifen, die auf einem Schwungrad (11) einer Testmaschine (1) rollen, die mindestens ein Paar Messstationen (12a, 12b) umfasst, bei welchem die folgenden Schritte ausgeführt werden:
- Schritt A: Mithilfe der Konstruktionsdaten eines Fahrzeugs (Dc) und eines dynamischen Gleichgewichtsmodells werden die Beziehungen (f₁, f₂, f₃, f₄, f₅, f₆) zwischen der Translationsgeschwindigkeit (Vx) und den Beschleunigungen (Axₜ, Ayₜ, Azₜ) im Schwerpunkt (T) des Fahrzeugs und den Längskräften (Fx), Querkräften (Fy) und den Laständerungen (Fz), die von jeder Achse auf das Fahrgestell des Fahrzeugs ausgeübt werden, der Verteilung der Längskräfte (Fx_{vg}, Fx_{vg}, Fx_{rg}, Fx_{rd}) und der Laständerungen (Fz_{vg}, Fz_{vd}, Fz_{rg}, Fz_{rd}), die vom Boden auf jedes der Räder ausgeübt werden, und dem Sturzwinkel (Ac) jedes der Räder bestimmt;
- Schritt B: Die Translationsgeschwindigkeit (Vx) und die Beschleunigungen (Axₜ, Ayₜ, Azₜ) im Schwerpunkt (T) des Fahrzeugs werden kontinuierlich gemessen und aufgezeichnet, wenn das Fahrzeug auf einer vorbestimmten Verschleißteststrecke fährt;
- Schritt C: An jeder der Messstationen (12a, 12b) der Testmaschine (1) werden die zwei Reifen angeordnet, die zu derselben Achse gehören, und
o mithilfe der Beziehungen (f₁, f₂, f₃, f₄, f₅, f₆), die im Schritt A bestimmt wurden, und in Abhängigkeit von den Werten der Translationsgeschwindigkeit (Vx) und der Längsbeschleunigung (Axₜ), Querbeschleunigung (Ayₜ) und vertikalen Beschleunigung (Azₜ) , die im Schritt B aufgezeichnet wurden, werden zu jedem Zeitpunkt die Werte der Längskraft (Fx), der Querkraft (Fy) und der Laständerung (Fz), die von der Achse (E) auf das Fahrgestell des Fahrzeugs ausgeübt werden, sowie die Werte des Sturzwinkels (Ac), von Laständerungen (Fz_{g}, Fz_{d}) und von Längskräften (Fx_{g}, Fx_{d}), die vom Boden auf jedes der Räder (W_{g}, W_{d}) der Achse (E) ausgeübt werden, bestimmt,
o es werden zu jedem Zeitpunkt an jedem der Räder (W_{g}, W_{d}) die Werte des Sturzwinkels (Ac) sowie die Werte der Längskräfte und vertikalen Kräfte (Fx_{g}, Fx_{d} und Fz_{g}, Fz_{d}), die zuvor berechnet wurden, angewendet, und
o es werden die Werte der Querkräfte (Fy_{g}, FY_{d}), die auf jedes der Räder (W_{g}, W_{d}) der Achse (E) einwirken, gemessen, und es wird der Schräglaufwinkel (Ad) jedes der Räder so variiert, dass die Summe der Querkräfte (Fy_{g}+Fy_{d}), die aus dem Schräglauf der Reifen resultieren, zu jedem Zeitpunkt gleich der Querkraft (Fy) ist, die auf den Mittelpunkt der Achse (E) ausgeübt wird, und dass für die Schräglaufdifferenz (δ(Ad)) zwischen den Rädern die aus den Merkmalen der Achse berechnete Parallelitätsabweichung eingehalten wird.

2. Testverfahren nach Anspruch 1, wobei der Wert der Längskraft, die von jeder Achse auf das Fahrgestell ausgeübt wird, aus dem dynamischen Gleichgewichtsmodell des Fahrzeugs unter der Einwirkung der Längsbeschleunigung (Ax₁), der Konstruktionsdaten (Dc) des Fahrzeugs, der Translationsgeschwindigkeit (Vx) und der vom Motor gelieferten Zugkräfte resultiert (*Fx* = *f*₁ (*Axₜ*, *Vx, Dc) ) .*

3. Testverfahren nach Anspruch 2, wobei die Längskräfte, die vom Boden auf jedes der Räder derselben Achse ausgeübt werden, zu jedem Zeitpunkt gleich sind (Fx_{g} = FX_{d}) .

4. Testverfahren nach Anspruch 2 oder Anspruch 3, wobei die Verteilung zwischen den Längskräften, die vom Boden auf jedes der vier Räder ausgeübt werden (Fx_{vg}, Fx_{vd}, Fx_{rg}, Fx_{rd}), aus einem speziellen Steuerungsgesetz resultiert, das in das dynamische Gleichgewichtsmodell des Fahrzeugs eingeführt wurde.

5. Testverfahren nach einem der vorhergehenden Ansprüche, wobei die Summe der Laständerungen (Fz_{g}+Fz_{d}), die vom Boden auf jedes der Räder (W_{g}, W_{d}) derselben Achse ausgeübt werden, aus dem dynamischen Gleichgewichtsmodell des Fahrzeugs beim Nicken unter der Einwirkung der Geschwindigkeit (Vx), der Längsbeschleunigung (Axₜ) und der Konstruktionsdaten (Dc) des Fahrzeugs resultiert (*Fz_{g}* + Fz_{d} *= f₂* (Axₜ, *Vx, Dc*) ) *.*

6. Testverfahren nach einem der vorhergehenden Ansprüche, wobei die Differenz der Laständerungen (Fz_{g}-Fz_{d}), die vom Boden auf jedes der Räder (W_{g}, W_{d}) derselben Achse ausgeübt werden, aus dem dynamischen Gleichgewichtsmodell des Fahrzeugs beim Wanken unter der Einwirkung der Querbeschleunigung (Ayₜ) und der Konstruktionsdaten (Dc) des Fahrzeugs resultiert (*Fz_{g}* - Fz_{d} *= f₃ (Ayₜ, Dc*))*.*

7. Testverfahren nach einem der vorhergehenden Ansprüche, wobei der Wert der Querkraft (Fy), die von jeder Achse auf das Fahrgestell ausgeübt wird, aus dem dynamischen Gleichgewichtsmodell des Fahrzeugs beim Gieren unter der Einwirkung der Querbeschleunigung (Ayₜ) und der Konstruktionsdaten (Dc) des Fahrzeugs resultiert (*Fy* = f₄ *(Ayₜ, Dc)).*

8. Testverfahren nach einem der vorhergehenden Ansprüche, wobei der Sturzwinkel (Ac) jedes Rades aus dem dynamischen Gleichgewichtsmodell des Fahrzeugs beim Wanken unter der Einwirkung der Querbeschleunigung (Ayₜ) und der Konstruktionsdaten (Dc) des Fahrzeugs resultiert (*Ac* = f₅ *(Ayₜ, Dc*)) *.*

9. Testverfahren nach Anspruch 8, wobei der Wert des Sturzwinkels (Ac) so korrigiert wird, dass dieser Winkel aus dem dynamischen Gleichgewichtsmodell resultiert, das um die Wirkung der elastischen Änderungen des Sturzes korrigiert ist, die durch die zu jedem Zeitpunkt am Schwungrad gemessenen Querkräfte (Fy_{g} und Fy_{d}) hervorgerufen werden *(Ac_{vd}* = *f'₅(Ayₜ, Fy_{vd}, Fy_{vg}*, *Dc*) und *Ac_{vg}* = *f'₅* (*Ayₜ, Fy_{vg}, Fy_{vd}, Dc)).*

10. Testverfahren nach einem der vorhergehenden Ansprüche, wobei die Schräglaufwinkel jedes der Räder derselben Achse so variiert werden, dass die Differenz (Ad_{g}-Ad_{d}) zwischen den Schräglaufwinkeln jedes der Räder gleich einem bekannten Wert (δ(Ad)) ist (*δ*(*Ad*) = *f₆(Dc)).*

11. Testverfahren nach Anspruch 10, wobei der Wert der Differenz zwischen den Schräglaufwinkeln jedes der Räder derselben Achse (δ(Ad)) zu jedem Zeitpunkt gleich null ist.

12. Testverfahren nach Anspruch 10, wobei der Wert der Differenz zwischen den Schräglaufwinkeln jedes der Räder derselben Achse (δ(Ad)) so korrigiert wird, dass diese Differenz (Ad_{g}-Ad_{d}) aus dem dynamischen Gleichgewichtsmodell resultiert, das um die Wirkung des Wertes der elastischen Änderungen des Radeinschlags korrigiert ist, die durch die Laständerungen (Fz_{vg}, Fz_{vd}) und die berechneten Längskräfte (Fx_{vg}, Fx_{vd}) und durch die zu jedem Zeitpunkt am Schwungrad gemessenen Querkräfte (Fy_{g} und Fy_{d}) hervorgerufen werden *(δ(Ad) = f'₆(Dc, F_{Xvg},* Fx_{vd}, *Fy_{vg}, Fy_{vd}, Fz_{vg}, Fz_{vd})* ) .

13. Testverfahren nach einem der vorhergehenden Ansprüche, wobei die Testmaschine (1) mit mehreren Paaren von Messstationen (12a, 12b) ausgerüstet ist und wobei durchgeführt werden:
- Tests von Reifensätzen, die auf der Vorder- und der Hinterachse desselben Fahrzeugs montiert sind,
- Tests von verschiedenen Reifensätzen, die zu derselben Achse desselben Fahrzeugs gehören,
- Tests von Reifensätzen, die auf derselben Achse desselben Fahrzeugs montiert sind und unterschiedliche Einstellungen der Konstruktionsdaten des Fahrzeugs Dc aufweisen,
- Tests von identischen Reifensätzen, die auf derselben Achse verschiedener Fahrzeuge montiert sind,
- Tests von verschiedenen Reifensätzen, die auf derselben Achse verschiedener Fahrzeuge montiert sind.

## Claims

1. Method for testing wear on tyres running on a rolling road (11) of a test machine (1) comprising at least one pair of measurement stations (12a, 12b), during which the following steps are carried out:
- Step A: with the aid of the construction data of a vehicle (Dc) and of a dynamic equilibrium model, the relationships (f₁, f₂, f₃, f₄, f₅, f₆) between the translational speed (Vx) and the accelerations (Axₜ, Ayₜ, Azₜ) at the centre of gravity of the vehicle (T), and the longitudinal forces (Fx), transverse forces (Fy) and the variations in loads (Fz) applied by each axle to the chassis of the vehicle, the distribution of the longitudinal forces (Fx_{vg}, Fx_{vd}, Fx_{rg}, Fx_{rd}) and of the variations in loads (Fz_{vg}, Fz_{vd}, Fz_{rg}, Fz_{rd}) applied by the ground to each of the wheels, and the camber angle (Ac) of each of the wheels are determined,
- Step B: the translational speed (Vx) and the accelerations (Axₜ, Ayₜ, Azₜ) at the centre of gravity (T) of the vehicle when the vehicle is travelling on a predetermined wear test course are measured and recorded continuously,
- Step C: the two tyres belonging to one and the same axle are disposed on each of the measurement stations (12a, 12b) of the test machine (1), and,
∘ with the aid of the relationships (f₁, f₂, f₃, f₄, f₅, f₆) determined in step A and depending on the values of translational speed (Vx) and longitudinal acceleration (Axₜ), transverse acceleration (Ayₜ) and vertical acceleration (Azₜ) recorded in step B, the values of the longitudinal force (Fx), of the transverse force (Fy) and of the variation in load (Fz) applied by the axle (E) to the chassis of the vehicle, and the values of camber angle (Ac), of variations in loads (Fz_{g}, Fz_{d}) and of longitudinal forces (Fx_{g}, Fx_{d}) applied by the ground to each of the wheels (W_{g}, W_{d}) of said axle (E) are determined at all times,
∘ the values of camber angle (Ac) and the values of the longitudinal and vertical forces (Fx_{g}, Fx_{d}, and Fz_{g}, Fz_{d}) previously calculated are applied to each of the wheels (W_{g}, W_{d}) at all times, and
∘ the values of the transverse forces (Fy_{g}, Fy_{d}) to which each of the wheels (W_{g}, W_{d}) of said axle (E) are subjected are measured and the drift angle (Ad) of each of the wheels is varied such that the sum of the transverse forces (Fy_{g}+Fy_{d}) resulting from the skewing of the tyres is equal at all times to the transverse force (Fy) applied to the centre of the axle (E) and such that the difference in drift (δ(Ad)) between the two wheels respects the variation in parallelism calculated on the basis of the characteristics of the axle.

2. Test method according to Claim 1, wherein the value of the longitudinal force applied by each axle to the chassis results from the dynamic equilibrium model of the vehicle under the effect of the longitudinal acceleration (Axₜ), the construction data (Dc) of the vehicle, the translational speed (Vx) and the tensile forces supplied by the engine *(Fx = f₁(AₓₜVₓ,D_{c})).*

3. Test method according to Claim 2, wherein the longitudinal forces applied by the ground to each of the wheels of one and the same axle are equal (Fx_{g}=Fx_{d}) at all times.

4. Test method according to Claim 2 or Claim 3, wherein the distribution between the longitudinal forces applied by the ground to each of the four wheels (Fx_{vg}, FX_{vd}, Fx_{rg}, Fx_{rd}) results from a specific control law introduced into the dynamic equilibrium model of the vehicle.

5. Test method according to any one of the preceding claims, wherein the sum of the variations in loads (Fz_{g}+Fz_{d}) applied by the ground to each of the wheels (W_{g}, W_{d}) of one and the same axle results from the pitch dynamic equilibrium model of the vehicle under the effect of the speed (Vx), the longitudinal acceleration (Axₜ) and the construction data (Dc) of the vehicle *(Fz_{g}* + *Fz_{d} = f₂(Axₜ,Vx,Dc)).*

6. Test method according to any one of the preceding claims, wherein the difference in the variations in loads (Fz_{g}-Fz_{d}) applied by the ground to each of the wheels (W_{g}, W_{d}) of one and the same axle results from the roll dynamic equilibrium model of the vehicle under the effect of the transverse acceleration (Ayₜ) and the construction data (Dc) of the vehicle (*Fz_{g}* - *Fz_{d} = f₃ (Ayₜ, Dc)).*

7. Test method according to any one of the preceding claims, wherein the value of the transverse force (Fy) applied by each axle to the chassis results from the yaw dynamic equilibrium model of the vehicle under the effect of the transverse acceleration (Ayₜ) and the construction data (Dc) of the vehicle *(Fy = f₄(Ayₜ, Dc)).*

8. Test method according to any one of the preceding claims, wherein the camber angle (Ac) of each wheel results from the roll dynamic equilibrium model of the vehicle under the effect of the transverse acceleration (Ayₜ) and the construction data (Dc) of the vehicle (*Ac* = *f₅ (Ayₜ, Dc)).*

9. Test method according to Claim 8, wherein the value of the camber angle (Ac) is corrected such that this angle results from the corrected dynamic equilibrium model of the effect of elastic variations in camber angle that are brought about by the transverse forces (Fy_{g} and Fy_{d}) measured at all times on the rolling road *(Ac_{vd} = f'₅(Ayₜ, Fy_{vd},Fy_{vg},Dc)* and *Ac_{vg}* = *f'* ₅ *(Ayₜ,* Fy_{vg}, *Fy_{vd}, Dc)).*

10. Test method according to any one of the preceding claims, wherein the drift angles of each of the wheels of one and the same axle are varied such that the difference (Ad_{g}-Ad_{d}) between the drift angles of each of the wheels is equal to a known value (δ(Ad)) (*δ*(*Ad*) = *f₆(Dc)).*

11. Test method according to Claim 10, wherein the value of the difference between the drift angles of each of the wheels of one and the same axle (δ(Ad)) is zero at all times.

12. Test method according to Claim 10, wherein the value of the difference between the drift angles of each of the wheels of one and the same axle (δ(Ad)) is corrected such that this difference (Ad_{g}-Ad_{d}) results from the corrected dynamic equilibrium model of the effect of the value of the elastic variations in steering angle that are brought about by the variations in load (Fz_{vg}, Fz_{vd}) and the longitudinal forces (Fx_{vg}, Fx_{vd}) calculated, and by the transverse forces (Fy_{g} and Fy_{d}) measured at all times on the rolling road (*δ*(*Ad*) = *f'₆(Dc, Fx_{vg}, Fx_{vd},* Fy_{vg}, *Fy_{vd}, Fz_{vg}, Fz_{vd}).*

13. Test method according to any one of the preceding claims, wherein the test machine (1) is equipped with a plurality of pairs (12a, 12b) of measurement stations and wherein the following are carried out:
- tests of sets of tyres mounted on the front and rear axles of one and the same vehicle,
- tests of different sets of tyres belonging to one and the same axle of one and the same vehicle,
- tests of sets of tyres mounted on the same axle of one and the same vehicle, involving different adjustments of the construction data of the vehicle Dc,
- tests of identical sets of tyres mounted on the same axle of different vehicles,
- tests of different sets of tyres mounted on the same axle of different vehicles.
